# EUROPEAN PATENT APPLICATION

(11) **EP 3 822 512 A1**
(43) Date of publication of application: **19.05.2021**
(21) Application number: 19855201.0
(22) Date of filing: 18.07.2019
(51) Int. Cl.: F16H 21/46, A61B 34/35, A61B 34/37, B25J 3/00

(54) **PARALLEL WIRE DEVICE, PARALLEL WIRE SYSTEM, MEDICAL ROBOT OPERATION DEVICE, MOBILE PROJECTION DEVICE, AND MOBILE IMAGING DEVICE**

(30) Priority: 27.08.2018 JP 2018158786
(71) Applicant: Sony Corporation, 108-0075 Tokyo (JP)
(72) Inventor: HONGO, Kazuo, Tokyo 108-0075 (JP); CONUS, William Alexandre, Tokyo 108-0075 (JP)
(74) Representative: D Young & Co LLP
(86) International application number: PCT/JP2019/028278
(87) International publication number: WO 2020/044838

(57) **Abstract**

A parallel wire device capable of translating a target object and rotating the target object in a wide movable range is provided.

The parallel wire device includes a movement unit, a rotational movement unit attached to the movement unit so as to be rotatable about at least one axis, a first parallel wire configured to translationally drive the movement unit, and a second parallel wire configured to rotationally drive the rotational movement unit. The rotational movement unit includes a parallel link, and the second parallel wire includes parallel wires configured to drive respective links constituting the parallel link.

## Description

### [Technical Field]

The technology disclosed in the present specification relates to a parallel wire device, a parallel wire system, an operating device for a medical robot, a mobile projecting device, and a mobile photographing device that translate and rotate a target object.

### [Background Art]

A parallel wires system and a parallel link system are known as driving systems with small inertia. In general, the parallel wire system has smaller inertia. These parallel mechanisms can be used to drive a controller operated by an operator on a master side and a device (end effector) at an output terminal on a slave side in a master-slave system, for example.

For example, a proposal has been made with regard to a tendon suspend platform robot of the parallel wire system, the robot including a platform suspended by a plurality of flexible tendons, and translating and rotating the platform by controlling the extension length of each of the flexible tendons (see PTL 1). In addition, a wearable force sense presenting device of the parallel wire system has been proposed (see PTL 2).

When a parallel wire mechanism with small inertia is applied to the controller operated by the operator on the master side, advantages are obtained in that a load is small and the operator does not become fatigued easily. When an application involving usage for a long period of time as in a case of a medical robot that performs endoscopic surgery or the like is considered, it is preferable that the controller have low inertia and that the load on the operator be reduced.

However, the parallel wire mechanism has a problem of a small rotational movable range. This is because wires interfere with each other during rotation. A wrist of a human has a movable range of approximately ±90 degrees, and the controller operated by a human preferably has a movable range corresponding to that of the wrist. Thus, it is difficult to apply the parallel wire mechanism having the small rotational movable range. In addition, another problem also occurs in that the wires and hands interfere with one another.

Meanwhile, a system in which equipment for photographing is supported by parallel wires in a ceiling-suspended manner and special photographing is performed in the air has already been put to practical use. In a case where a parallel wire device is used to thus drive a heavy object, the weight of the parallel wire device is preferably reduced as much as possible in preparation for a danger at a time of falling.

### [Citation List]

### [Patent Literature]

[PTL 1]
   Japanese Translation of PCT International Application Publication No. JP-T-H09-500337
[PTL 2]
   JP 2016-207005A
[PTL 3]
   WO2017/130562
[PTL 4]
   JP 2000-79586A

### [Summary]

### [Technical Problems]

It is an object of the technology disclosed in the present specification to provide a parallel wire device, a parallel wire system, an operating device for a medical robot, a mobile projecting device, and a mobile photographing device that can translate a target object and rotate the target object in a wide movable range.

### [Solution to Problems]

The technology disclosed in the present specification has been made in consideration of the above-described problems. According to a first aspect of the technology disclosed in the present specification, there is provided a parallel wire device including a movement unit, a rotational movement unit attached to the movement unit so as to be rotatable about at least one axis, a first parallel wire configured to translationally drive the movement unit, and a second parallel wire configured to rotationally drive the rotational movement unit.

The rotational movement unit has two or more degrees of rotational freedom with respect to the movement unit, and the second parallel wire includes a parallel wire configured to rotationally drive the rotational movement unit about one axis and a parallel wire configured to rotationally drive the rotational movement unit about another axis.

The rotational movement unit includes a serial link, and the second parallel wire includes a parallel wire configured to drive the serial link. Alternatively, the rotational movement unit includes a parallel link, and the second parallel wire includes parallel wires configured to drive respective links constituting the parallel link.

In addition, according to a second aspect of the technology disclosed in the present specification, there is provided a parallel wire system including a movement unit, a rotational movement unit attached to the movement unit so as to be rotatable about at least one axis, a first parallel wire configured to translationally drive the movement unit, a second parallel wire configured to rotationally drive the rotational movement unit, and a plurality of actuators configured to drive respective wires included in the first parallel wire and the second parallel wire.

However, a "system" referred to herein refers to a logical set of a plurality of devices (or functional modules implementing specific functions), and no particular distinction is made as to whether or not devices and function modules are present within a single casing.

In addition, according to a third aspect of the technology disclosed in the present specification, there is provided an operating device for a medical robot of a master-slave system, the operating device being for operating a medical instrument attached to a slave side from a master side, the operating device including a movement unit, a rotational movement unit attached to the movement unit so as to be rotatable about at least one axis, an operating unit for an operator, the operating unit being attached to the rotational movable unit, a first parallel wire configured to translationally drive the movement unit, and a second parallel wire configured to rotationally drive the rotational movement unit.

In addition, according to a fourth aspect of the technology disclosed in the present specification, there is provided a mobile projecting device including a movement unit, a rotational movement unit attached to the movement unit so as to be rotatable about at least one axis, a projecting device attached to the rotational movable unit, a first parallel wire configured to translationally drive the movement unit, and a second parallel wire configured to rotationally drive the rotational movement unit.

In addition, according to a fifth aspect of the technology disclosed in the present specification, there is provided a mobile photographing device including a movement unit, a rotational movement unit attached to the movement unit so as to be rotatable about at least one axis, a camera attached to the rotational movable unit, a first parallel wire configured to translationally drive the movement unit, and a second parallel wire configured to rotationally drive the rotational movement unit.

### [Advantageous Effects of Invention]

According to the technology disclosed in the present specification, it is possible to provide a parallel wire device, a parallel wire system, an operating device for a medical robot, a mobile projecting device, and a mobile photographing device that can translate a target object and rotate the target object in a wide movable range.

It is to be noted that the effects described in the present specification are merely illustrative, and that the effects of the present invention are not limited thereto. In addition, the present invention may further produce additional effects other than the above-described effects.

Still other objects, features, and advantages of the technology disclosed in the present specification will become apparent from more detailed description based on embodiments to be described later and the accompanying drawings.

### [Brief Description of Drawings]

[FIG. 1]

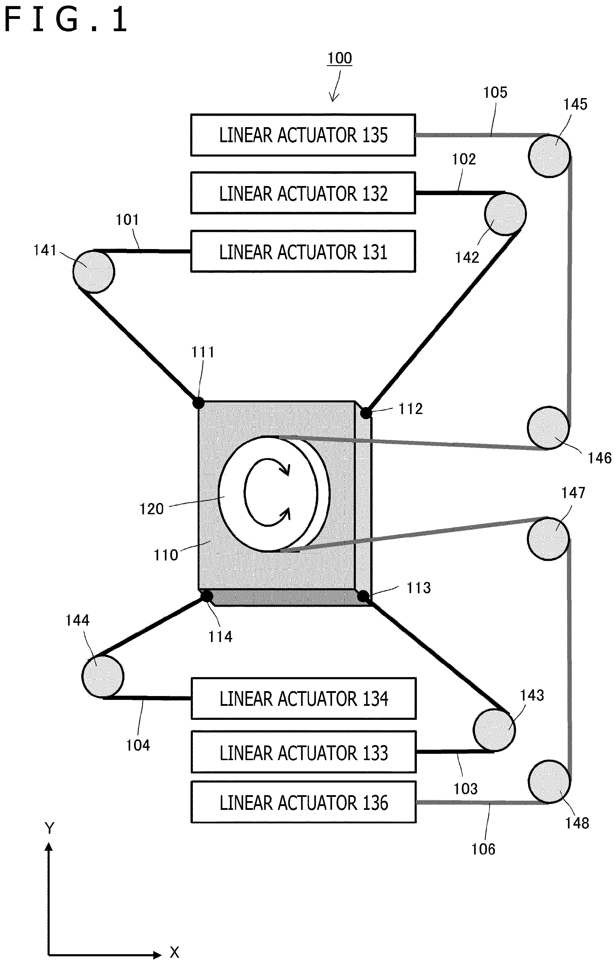

   FIG. 1 is a diagram depicting a configuration of a parallel wire device 100.
[FIG. 2]

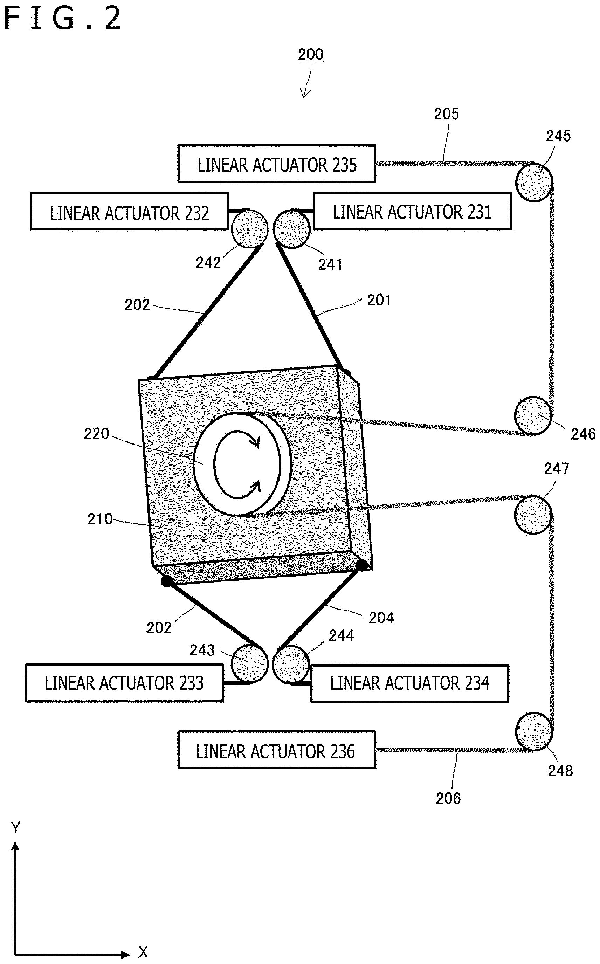

   FIG. 2 is a diagram depicting a configuration of a parallel wire device 200.
[FIG. 3]

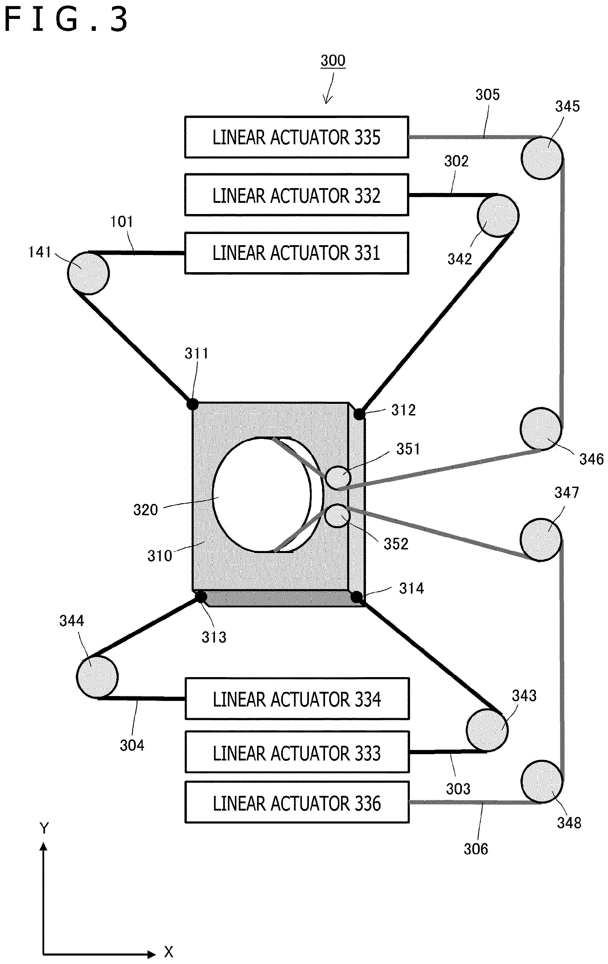

   FIG. 3 is a diagram depicting a configuration of a parallel wire device 300.
[FIG. 4]

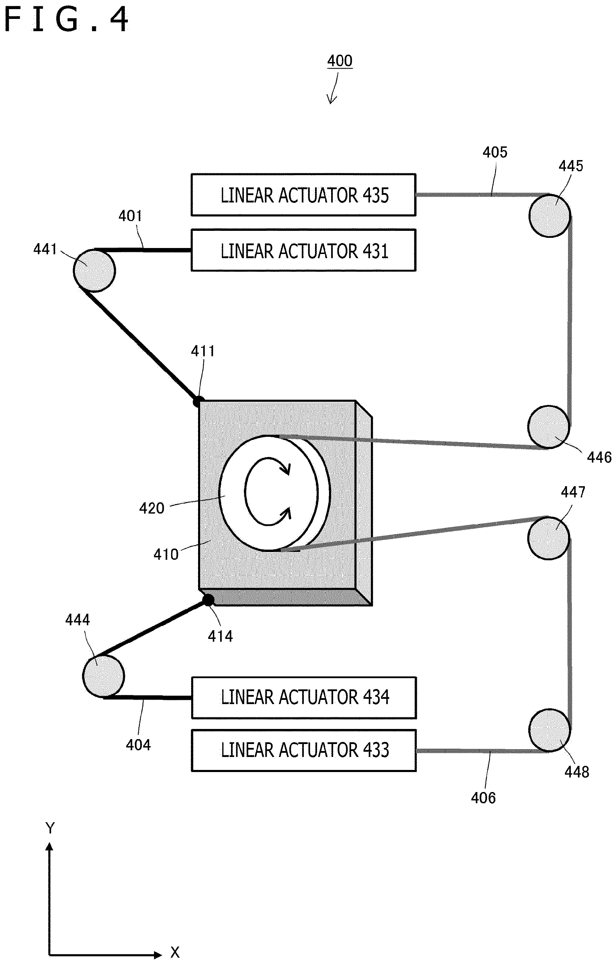

   FIG. 4 is a diagram depicting a configuration of a parallel wire device 400.
[FIG. 5]

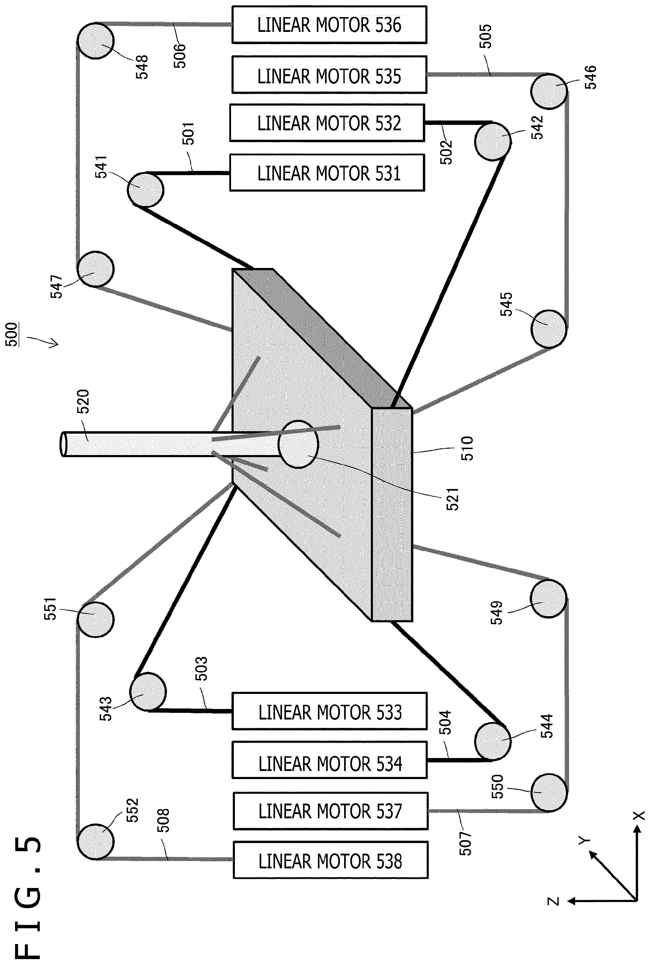

   FIG. 5 is a diagram depicting a configuration of a parallel wire device 500.
[FIG. 6]

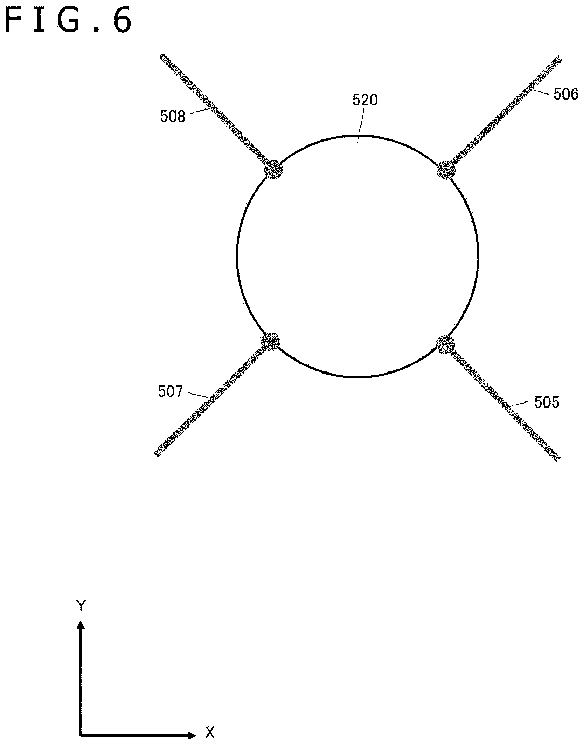

   FIG. 6 is a diagram depicting a state in which a rotational movement unit 520 of the parallel wire device 500 is viewed from above.
[FIG. 7]

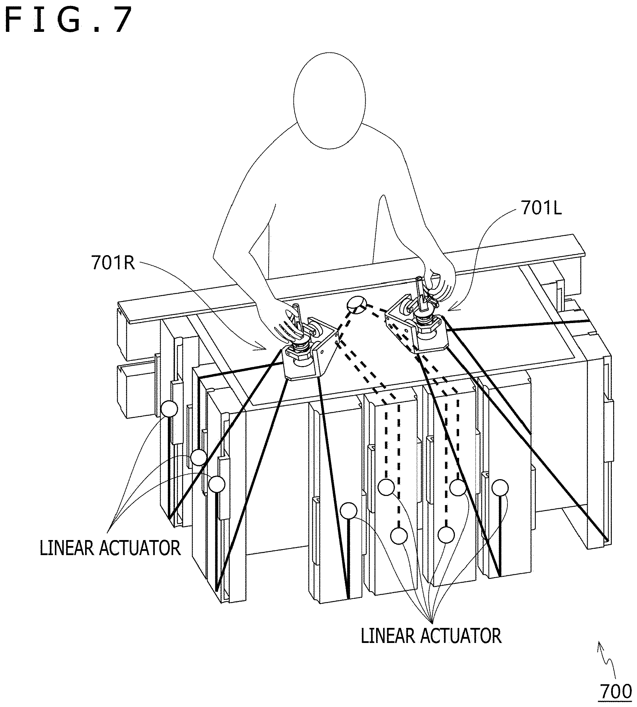

   FIG. 7 is a diagram depicting a configuration example of a master device 700 to which a parallel wire system is applied.
[FIG. 8]

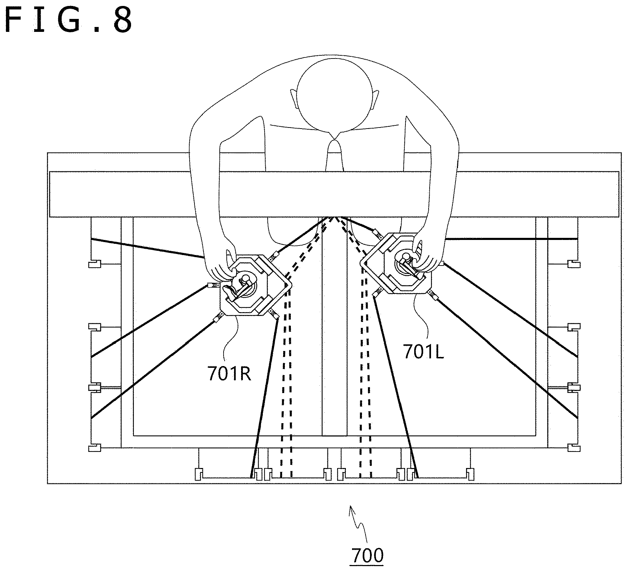

   FIG. 8 is a diagram depicting the configuration example of the master device 700 to which the parallel wire system is applied.
[FIG. 9]

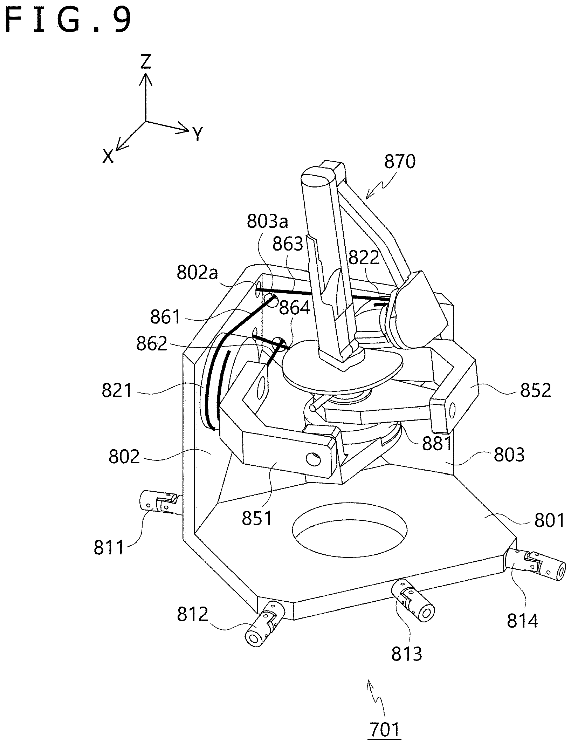

   FIG. 9 is a diagram depicting a controller 701.
[FIG. 10]

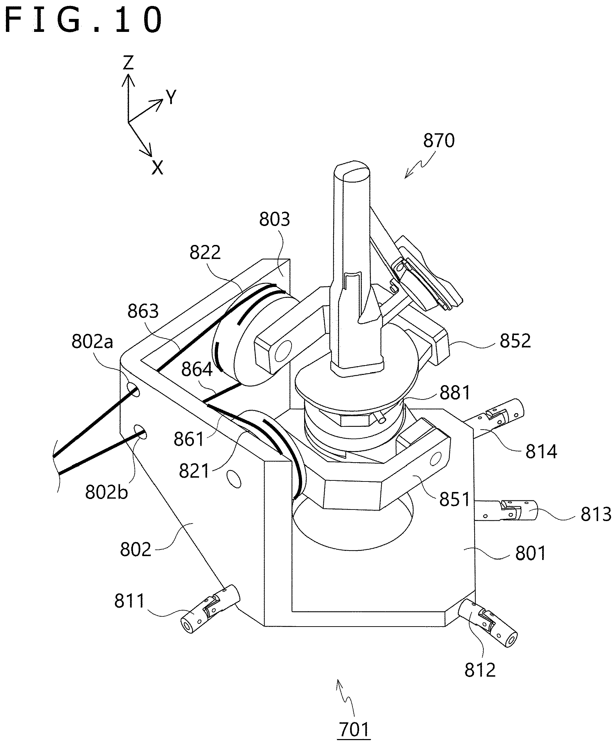

   FIG. 10 is a diagram depicting the controller 701.
[FIG. 11]

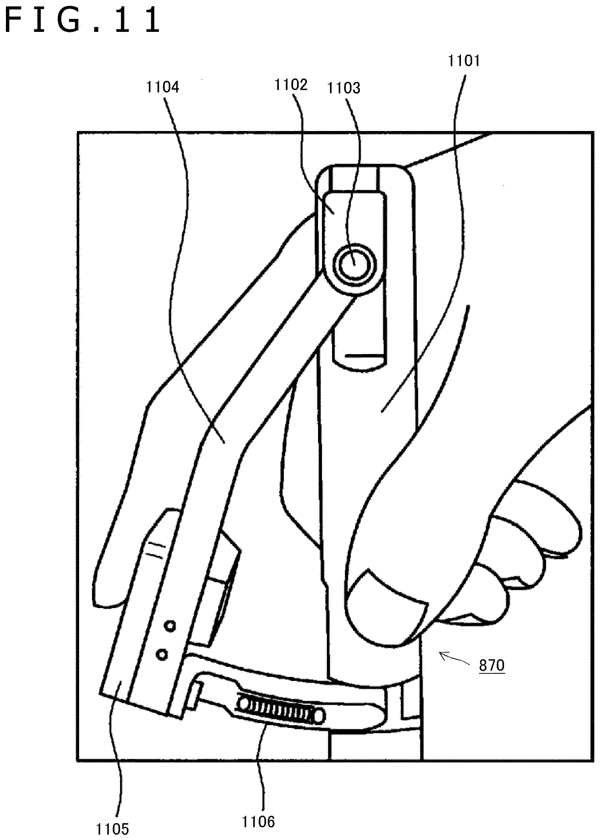

   FIG. 11 is a diagram depicting a state in which an operator is using a gripping force sense presenting device 870.
[FIG. 12]

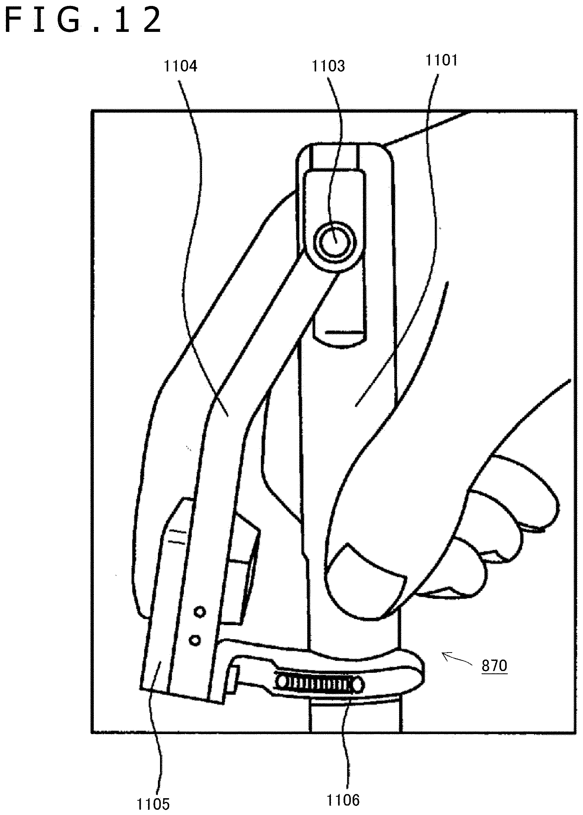

   FIG. 12 is a diagram depicting a state in which the operator is using the gripping force sense presenting device 870.
[FIG. 13]

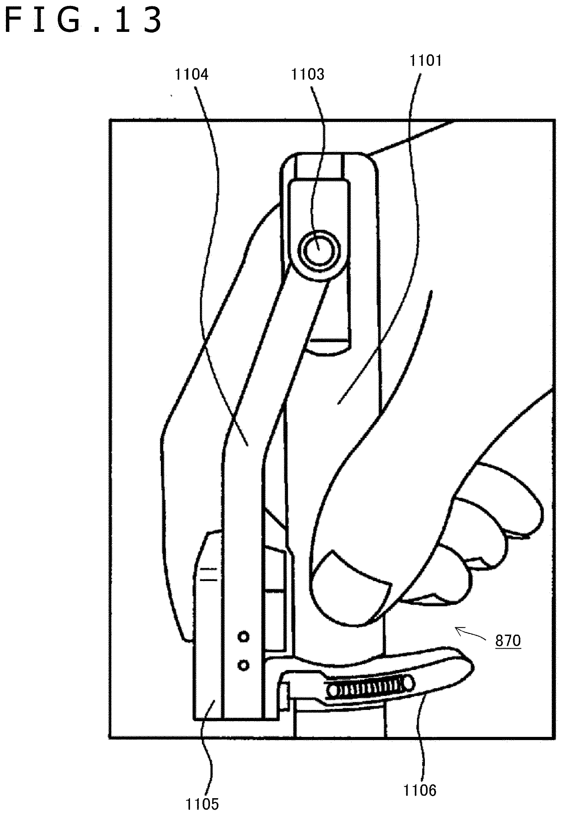

   FIG. 13 is a diagram depicting a state in which the operator is using the gripping force sense presenting device 870.
[FIG. 14]

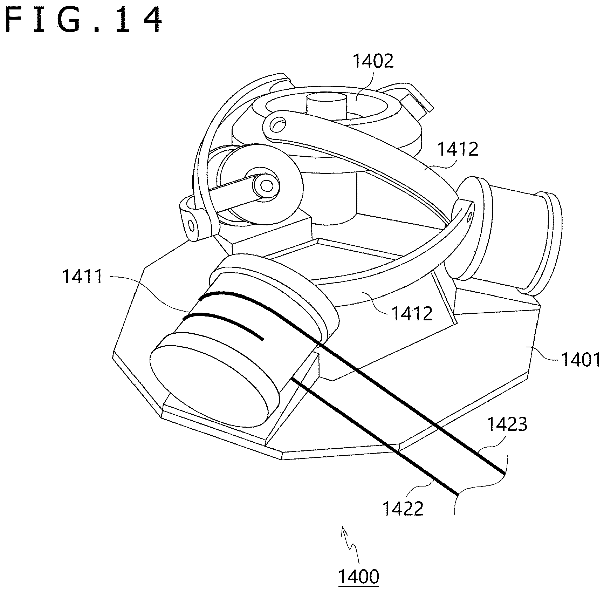

   FIG. 14 is a diagram depicting a configuration example of a spherical parallel link device.
[FIG. 15]

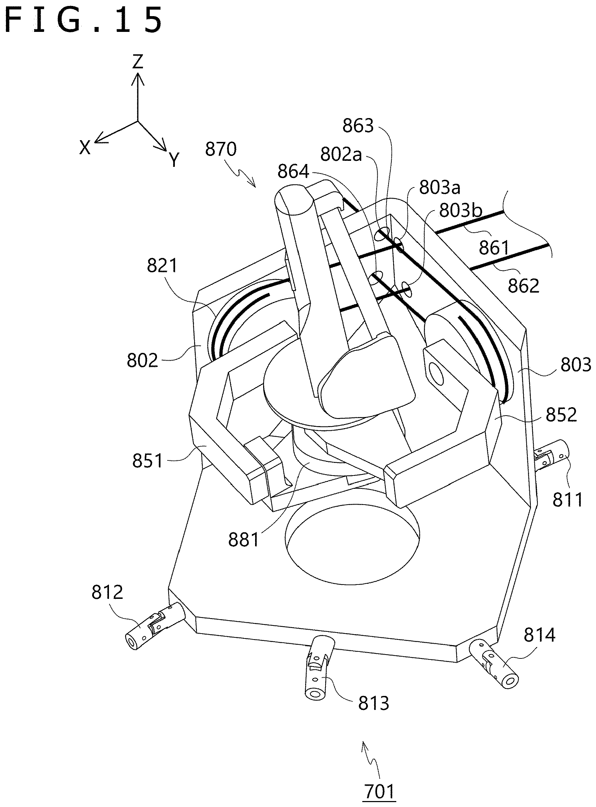

   FIG. 15 is a diagram depicting the controller 701.

### [Description of Embodiments]

In the following, five basic configuration examples of parallel wire devices to which the technology disclosed in the present specification is applied are illustrated as a first embodiment in FIGS. 1 to 5 and will be described with reference to the respective drawings. In addition, an embodiment in which a parallel wire system according to the technology disclosed in the present specification is applied to a controller of a master device of a master-slave system will be described as a second embodiment with reference mainly to FIGS. 7 to 10. In addition, a modification of a parallel link device driven by using a parallel wire device according to the technology disclosed in the present specification will be described as a third embodiment with reference to FIG. 14. Moreover, a range of application of the parallel wire devices disclosed in the present specification will be mentioned as a fourth embodiment.

### First Embodiment

FIG. 1 schematically illustrates a basic configuration example of a parallel wire device 100 to which the technology disclosed in the present specification is applied. A paper plane will be set as an XY plane, and a Z-axis will be set in a direction perpendicular to the paper plane. In addition, for the convenience of description, suppose that the parallel wire device 100 illustrated in FIG. 1 has a total of three degrees of freedom including degrees of translational freedom in two X- and Y-directions and a degree of rotational freedom about one axis, that is, the Z-axis.

The parallel wire device 100 includes six parallel wires 101 to 106 and a movement unit 110 supported by these wires 101 to 106. In addition, a rotational movement unit 120 is attached to an upper surface of the movement unit 110 so as to be rotatable at least about the Z-axis with respect to the movement unit 110. Incidentally, the movement unit 110 is assumed to be supported slidably on a flat surface, for example, on a board (not illustrated) or the like.

The wire 101 has a distal end portion fixed to an end portion 111 of the movement unit 110, and has a proximal portion attached to a linear actuator 131. A length of the wire 101 can be controlled by driving of the linear actuator 131. In the illustrated example, a driving direction of the linear actuator 131 is changed by winding the wire 101 around a direction changing pulley 141, and the distal end portion of the wire 101 is attached to the end portion 111. The distal end portion of the wire 101 is preferably joined to the end portion 111 by a joint that changes in angle freely, such as a universal joint or the like.

In addition, the wire 102 has a distal end portion fixed to an end portion 112 of the movement unit 110, and has a proximal portion attached to a linear actuator 132. A length of the wire 102 can be controlled by driving of the linear actuator 132. However, a driving direction of the linear actuator 132 is changed by winding the wire 102 around a direction changing pulley 142, and the distal end portion of the wire 102 is attached to the end portion 112. The distal end portion of the wire 102 is preferably joined to the end portion 112 by a joint that changes in angle freely, such as a universal joint or the like.

In addition, the wire 103 has a distal end portion fixed to an end portion 113 of the movement unit 110, and has a proximal portion attached to a linear actuator 133. A length of the wire 103 can be controlled by driving of the linear actuator 133. However, a driving direction of the linear actuator 133 is changed by winding the wire 103 around a direction changing pulley 143, and the distal end portion of the wire 103 is attached to the end portion 113. The distal end portion of the wire 103 is preferably joined to the end portion 113 by a joint that changes in angle freely, such as a universal joint or the like.

In addition, the wire 104 has a distal end portion fixed to an end portion 114 of the movement unit 110, and has a proximal portion attached to a linear actuator 134. A length of the wire 104 can be controlled by driving of the linear actuator 134. However, a driving direction of the linear actuator 134 is changed by winding the wire 104 around a direction changing pulley 144, and the distal end portion of the wire 104 is attached to the end portion 114. The distal end portion of the wire 104 is preferably joined to the end portion 114 by a joint that changes in angle freely, such as a universal joint or the like.

A distal end portion of the wire 105 is wound around and fixed to a side surface portion of the rotational movement unit 120, and a distal end portion of the wire 106 is wound around and fixed to the side surface portion of the rotational movement unit 120 in an opposite direction from the wire 105. Then, a proximal portion of the wire 105 is attached to a linear actuator 135, and a proximal portion of the wire 106 is attached to a linear actuator 136. The lengths of the wires 105 and 106 can be respectively controlled by driving of the respective linear actuators 135 and 136. However, a driving direction of the linear actuator 135 is changed by winding the wire 105 around direction changing pulleys 145 and 146, and the wire 105 is then wound around the side surface portion of the rotational movement unit 120. In addition, a driving direction of the linear actuator 136 is changed by winding the wire 106 around direction changing pulleys 147 and 148, and the wire 106 is then wound around the side surface portion of the rotational movement unit 120.

Suppose that each of the linear actuators 131 to 136 is collectively controlled by a control unit not illustrated.

The four wires 101 to 104 are parallel wires for translating the movement unit 110 in an XY plane. The control unit changes the length of each of the wires 101 to 104 by synchronously driving the linear actuators 131 to 134 at the proximal portions of the respective wires 101 to 104. The movement unit 110 can be thereby translated in the XY plane. It is also possible to rotate the movement unit 110 about the Z-axis in a certain movable range in an XYZ coordinate system by synchronously changing the lengths of the respective wires 101 to 104.

Specifically, the movement unit 110 is moved in a positive Y-direction by pulling the wires 101 and 102 by the linear actuators 131 and 132, and extending the wires 103 and 104 by the linear actuators 133 and 134 so as to balance with the pulling of the wires 101 and 102. The movement unit 110 is moved in a negative Y-direction by pulling the wires 103 and 104 by the linear actuators 133 and 134 and extending the wires 101 and 102 by the linear actuators 131 and 132 so as to balance with the pulling of the wires 103 and 104. In a case where the linear actuators are of a wire winding type, the wires are pulled by being wound, and the wires are extended by being unwound (the same is true in the following).

Meanwhile, the movement unit 110 is moved in a negative X-direction by pulling the wires 101 and 104 by the linear actuators 131 and 134 and extending the wires 102 and 103 by the linear actuators 132 and 133 so as to balance with the pulling of the wires 101 and 104. The movement unit 110 is moved in a positive X-direction by pulling the wires 102 and 103 by the linear actuators 132 and 133 and extending the wires 101 and 104 by the linear actuators 131 and 134 so as to balance with the pulling of the wires 102 and 103.

In addition, the two wires 105 and 106 are parallel wires for rotational movement of the rotational movement unit 120 on the movement unit 110 about the Z-axis. The rotational movement unit 120 can be rotated about the Z-axis with respect to the movement unit 110 (or in the XYZ coordinate system) when the control unit shortens the length of one of the two wires 105 and 106 and extends the other by such a length that the two wires are balanced by synchronously driving the linear actuators 135 and 136.

Specifically, the rotational movement unit 120 can be rotated clockwise in FIG. 1 by pulling the wire 105 by the linear actuator 135 and extending the wire 106 by the linear actuator 136 so as to balance with the pulling of the wire 105. Conversely, the rotational movement unit 120 can be rotated counterclockwise in FIG. 1 by pulling the wire 106 by the linear actuator 136 and extending the wire 105 by the linear actuator 135 so as to balance with the pulling of the wire 106.

Incidentally, in addition to the translation of the movement unit 110 in the XY plane, the movement unit 110 can be rotated about the Z-axis to a certain extent by changing the lengths of the respective wires 101 to 104. Hence, a wider rotational movable range can be secured by utilizing the functions of rotating both the movement unit 110 and the rotational movement unit 120.

In FIG. 1, the wires 101 to 104 provided in parallel to translate the movement unit 110 are depicted in black, and the wires 105 and 106 provided in parallel to rotate the rotational movement unit 120 are depicted in gray.

The linear actuators 131 to 136 can be, for example, formed by a ball screw, a shaft motor, a linear motor, a combination of a motor, a gear, and a rack type linear motion structure, or the like. However, the linear actuators 131 to 136 do not need to be linear actuators as long as the linear actuators 131 to 136 are capable of performing the operations of shortening and lengthening the lengths of the respective wires 101 to 106. For example, the linear actuators can be replaced by combining a rotary motor and a mechanism that winds a wire by rotation of the motor.

In the parallel wire device 100 formed by attaching the four wires 101 to 104 for translation and the two wires 105 and 106 for rotation to the movement unit 110 provided with the rotational movement unit 120, the arrangement of the wires 101 to 106 and the linear actuators 131 to 136 that drive the respective wires 101 to 106 is not limited to the configuration example illustrated in FIG. 1. In addition, the number and arrangement of the direction changing pulleys 141, 142, ... around which the respective wire 105 to 106 are wound are not limited to the configuration example illustrated in FIG. 1 either. It suffices to determine the arrangement of the linear actuators 131 to 136 and the direction changing pulleys 141, 142, ... in consideration of the movable range and ease of movement of the movement unit 110 and the rotational movement unit 120, interferences with other members not illustrated within the device 100, and the like.

The wires 101 to 106 used in the parallel wire device 100 according to the present embodiment can be fabricated by using, for example, a metallic string (a wire rope including a stranded wire manufactured of stainless steel or the like) or a chemical fiber. The metallic string has an advantage of being not elongated easily. In addition, while there is a risk of the chemical fiber being elongated easily, the chemical fiber has an advantage of being conforming easily. In addition, not all of the wires to be used necessarily needs to uniformly include an identical material.

FIG. 2 schematically illustrates an example of a configuration of another parallel wire device 200 to which the technology disclosed in the present specification is applied. However, in FIG. 2, XYZ axes are set in a similar manner to FIG. 1, and the parallel wire device 200 has a total of three degrees of freedom including degrees of translational freedom in two X- and Y-directions and a degree of rotational freedom about one axis, that is, the Z-axis.

The parallel wire device 200 includes six parallel wires 201 to 206 and a movement unit 210 supported by these wires 201 to 206. In addition, a rotational movement unit 220 rotatable about the Z-axis is attached to an upper surface of the movement unit 210. Incidentally, the movement unit 210 is assumed to be supported slidably on a flat surface, for example, on a board (not illustrated) or the like.

The parallel wire device 200 is similar to the parallel wire device 100 illustrated in FIG. 1 in that the wires 201 to 206 are respectively driven by linear actuators 231 to 236, in that the wires 201 to 206 are wound around direction changing pulleys 241 to 248, and in that the movement unit 210 is moved by the wires 201 to 204 and the rotational movement unit 220 is rotated by the wires 205 and 206.

A main difference between the parallel wire device 200 and the parallel wire device 100 is a method of attaching the wires 201 to 204 to the movement unit 210. Then, for the convenience of attaching the wires 201 to 204 to the movement unit 210, the arrangement of the linear actuators 231 to 234 driving the respective wires 201 to 204 and the direction changing pulleys 241 to 244 around which the respective wires 201 to 204 are wound is also changed.

Then, the parallel wire device 200 illustrated in FIG. 2 is configured such that the movement unit 210 is rotated easily by driving the wires 201 to 204, and naturally the rotational movement unit 220 is also rotated easily by driving the wires 201 to 204. Hence, an even wider rotational movable range than that of the parallel wire device 100 illustrated in FIG. 1 can be secured by utilizing the functions of rotating both the movement unit 210 and the rotational movement unit 220.

Specifically, the movement unit 210 can be rotated clockwise in FIG. 2 by pulling the wires 201 and 203 by the linear actuators 231 and 233 and extending the wires 202 and 204 by the linear actuators 232 and 234 so as to balance with the pulling of the wires 201 and 203. Conversely, the movement unit 210 can be rotated counterclockwise in FIG. 2 by pulling the wires 202 and 204 by the linear actuators 232 and 234 and extending the wires 201 and 203 by the linear actuators 231 and 233 so as to balance with the pulling of the wires 202 and 204.

Incidentally, the two wires 205 and 206 are parallel wires for rotational movement of the rotational movement unit 220 on the movement unit 210 about the Z-axis. The rotational movement unit 220 can be rotated about the Z-axis with respect to the movement unit 210 (or in the XYZ coordinate system) by shortening the length of one of the two wires 205 and 206 and extending the other by such a length that the two wires are balanced by synchronously driving the linear actuators 235 and 236.

FIG. 3 schematically illustrates an example of a configuration of yet another parallel wire device 200 to which the technology disclosed in the present specification is applied. However, in FIG. 3, XYZ axes are set in a similar manner to FIG. 1, and a parallel wire device 300 has a total of three degrees of freedom including degrees of translational freedom in two X- and Y-directions and a degree of rotational freedom about one axis, that is, the Z-axis.

The parallel wire device 300 includes six parallel wires 301 to 306, and a movement unit 310 supported by these wires 301 to 306. In addition, a rotational movement unit 320 rotatable about the Z-axis is attached to an upper surface of the movement unit 310. Incidentally, the movement unit 310 is assumed to be supported slidably on a flat surface, for example, on a board (not illustrated) or the like.

The parallel wire device 300 is similar to the parallel wire device 100 illustrated in FIG. 1 in that the wires 301 to 306 are driven by the respective linear actuators 331 to 336, in that the wires 301 to 306 are wound around direction changing pulleys 341 to 348, and in that the movement unit 310 is moved by the wires 301 to 304 and the rotational movement unit 320 is rotated by the wires 305 and 306.

A main difference between the parallel wire device 300 and the parallel wire device 100 lies in that the wires 305 and 306 for rotation-driving the rotational movement unit 320 are respectively wound around direction changing pulleys 351 and 352 each disposed on the movement unit 310, and thereafter, distal end portions of the respective wires 305 and 306 are wound around side surface portions of the rotational movement unit 320 in opposite directions from each other.

Even when the position or attitude of the movement unit 310 changes as a result of the movement unit 310 being translated by the driving of the wires 301 to 304 or of the rotational movement unit 320 being rotated or when the relative position of the movement unit 310 with respect to the direction changing pulleys 346 and 347 changes, the wires 305 and 306 can always be in contact with the side surface of the rotational movement unit 320 at fixed contact positions via the direction changing pulleys 351 and 352. Hence, the rotational movement unit 320 always receives the tensions of the wires 305 and 306 from the same directions. Such a configuration can widen a movable range in which the rotational movement unit 320 is rotated about the Z-axis.

Needless to say, in addition to the translation of the movement unit 310 in the XY plane, the movement unit 310 can be rotated about the Z-axis to a certain extent by changing the lengths of the respective wires 301 to 304. Hence, a wider rotational movable range can be secured by utilizing the functions of rotating both the movement unit 310 and the rotational movement unit 320.

FIG. 4 schematically illustrates an example of a configuration of yet another parallel wire device 200 to which the technology disclosed in the present specification is applied. However, in FIG. 4, XYZ axes are set in a similar manner to FIG. 1, and a parallel wire device 400 has a total of three degrees of freedom including degrees of translational freedom in two X- and Y-directions and a degree of rotational freedom about one axis, that is, the Z-axis.

The parallel wire device 400 includes four parallel wires 401, 404, 405, and 406 and a movement unit 410 supported by these wires 401, .... In addition, a rotational movement unit 420 rotatable about the Z-axis is attached to an upper surface of the movement unit 410. Incidentally, the movement unit 410 is assumed to be supported slidably on a flat surface, for example, on a board (not illustrated) or the like.

The wires 401 and 404 are parallel wires mainly for the translation of the movement unit 410. A distal end portion of the wire 401 is fixed to an end portion 411 of the movement unit 410, the wire 401 is wound around a direction changing pulley 441, and thereafter, a proximal portion of the wire 401 is attached to a linear actuator 431. In addition, a distal end portion of the wire 404 is fixed to an end portion 414 of the movement unit 410, the wire 404 is wound around a direction changing pulley 444, and thereafter a proximal portion of the wire 404 is attached to a linear actuator 434.

The wires 405 and 406 are parallel wires that rotate the rotational movement unit 420 and are also used for the translation of the movement unit 410. A distal end portion of the wire 405 is wound around and fixed to a side surface portion of the rotational movement unit 420, the wire 405 is wound around direction changing pulleys 445 and 446, and thereafter, a proximal portion of the wire 405 is attached to a linear actuator 435. In addition, a distal end portion of the wire 406 is wound around and fixed to a side surface portion of the rotational movement unit 420, the wire 406 is wound around direction changing pulleys 447 and 448, and thereafter, a proximal portion of the wire 406 is attached to a linear actuator 436.

The movement unit 410 is moved in a negative X-direction by pulling the wires 401 and 404 by the linear actuators 431 and 434 and extending the wires 405 and 406 by the linear actuators 435 and 436 so as to balance with the pulling of the wires 401 and 404. Conversely, the movement unit 410 is moved in a positive X-direction by pulling the wires 405 and 406 by the linear actuators 435 and 436 and extending the wires 401 and 404 by the linear actuators 431 and 434 so as to balance with the pulling of the wires 405 and 406.

In addition, the movement unit 410 is moved in a positive Y-direction by pulling the wires 401 and 405 by the linear actuators 431 and 435 and extending the wires 405 and 406 by the linear actuators 435 and 436 so as to balance with the pulling of the wires 401 and 405. Conversely, the movement unit 410 is moved in a negative Y-direction by pulling the wires 404 and 406 by the linear actuators 434 and 436 and extending the wires 401 and 405 by the linear actuators 431 and 435 so as to balance with the pulling of the wires 404 and 406.

In addition, the rotational movement unit 420 can be rotated clockwise in FIG. 4 by pulling the wire 405 by the linear actuator 435 and extending the wire 406 by the linear actuator 436 so as to balance with the pulling of the wire 405. On the other hand, the rotational movement unit 420 can be rotated counterclockwise in FIG. 4 by pulling the wire 406 by the linear actuator 436 and extending the wire 405 by the linear actuator 435 so as to balance with the pulling of the wire 406.

A main difference of the parallel wire device 400 illustrated in FIG. 4 from the parallel wire devices 100, 200, and 300 respectively illustrated in FIGS. 1 to 3 lies in that the wires 405 and 406 for the rotation of the rotational movement unit 420 are also used as wires for the translation of the movement unit 410. As described above, when one of the wires 405 and 406 for the rotation of the rotational movement unit 420 is pulled and the other is extended, the rotational movement unit 420 can be rotated, whereas the movement unit 410 can be translated by pulling or extending the wires 405 and 406 at the same time. That is, the rotational movement unit 420 is rotated by using a difference between changes in length of the wire 405 and the wire 406, and a sum of changes in length of the wire 405 and the wire 406 is used to translate the movement unit 410. As is understood from a comparison of FIG. 4 with FIGS. 1 to 3, a total number of wires and the number of linear actuators can be reduced by using the pair of wires for both rotation and translation. However, the movable range of rotation of the movement unit 410 is decreased.

FIG. 5 schematically illustrates an example of a configuration of yet another parallel wire device 200 to which the technology disclosed in the present specification is applied.

A parallel wire device 500 includes eight parallel wires 501 to 508 and a movement unit 510 supported by these wires 501 to 508. In order to simplify the drawing, the movement unit 510 forms a simple flat plate shape. Then, in FIG. 5, XY axes are set on a plane parallel with this flat plate, and a Z-axis is set in a direction perpendicular to the XY plane. Incidentally, the movement unit 510 is assumed to be supported slidably on a flat surface, for example, on a board (not illustrated) or the like.

The four wires 501 to 504 are parallel wires for translating the movement unit 510. A distal end portion of the wire 501 is fixed to an end portion 511 of the movement unit 510, the wire 501 is wound around a direction changing pulley 541, and thereafter, a proximal portion of the wire 501 is attached to a linear actuator 531. In addition, a distal end portion of the wire 502 is fixed to an end portion of the movement unit 510, the wire 502 is wound around a direction changing pulley 542, and thereafter, a proximal portion of the wire 502 is attached to a linear actuator 532. In addition, a distal end portion of the wire 503 is fixed to an end portion of the movement unit 510, the wire 503 is wound around a direction changing pulley 543, and thereafter, a proximal portion of the wire 503 is attached to a linear actuator 533. In addition, a distal end portion of the wire 504 is fixed to an end portion of the movement unit 510, the wire 504 is wound around a direction changing pulley 544, and thereafter, a proximal portion of the wire 504 is attached to a linear actuator 534. A mechanism for translating the movement unit 510 by using the four parallel wires is similar to that of the parallel wire device 100 illustrated in FIG. 1, and therefore, detailed description thereof will be omitted in the following.

The parallel wire device 500 greatly differs from the parallel wire devices 100 to 400 respectively illustrated in FIGS. 1 to 4 in that a rotational movement unit 520 having degrees of rotational freedom about at least two axes among the XYZ axes is attached to the upper surface of the movement unit 510. In the example illustrated in FIG. 5, the rotational movement unit 520 is a rod-shaped structure, and a connecting portion 521 as a base portion of the rotational movement unit 520 is formed by using a ball joint or a universal joint, for example, and imparts degrees of rotational freedom about at least two axes to the rotational movement unit 520.

The four wires 505 to 508 are parallel wires for rotating the rotational movement unit 520 about at least two axes with respect to the movement unit 510. A distal end portion of the wire 505 is fixed to the side surface of the rod-shaped rotational movement unit 520, passed through the lower surface side of the movement unit 510 via a through insertion hole drilled in the movement unit 510, and further wound around direction changing pulleys 545 and 546, and thereafter, a proximal portion of the wire 505 is attached to a linear actuator 535. In addition, a distal end portion of the wire 506 is fixed to the side surface of the rod-shaped rotational movement unit 520, passed through the lower surface side of the movement unit 510 via a through insertion hole drilled in the movement unit 510, and further wound around direction changing pulleys 547 and 548, and thereafter, a proximal portion of the wire 506 is attached to a linear actuator 536. In addition, a distal end portion of the wire 507 is fixed to the side surface of the rod-shaped rotational movement unit 520, passed through the lower surface side of the movement unit 510 via a through insertion hole drilled in the movement unit 510, and further wound around direction changing pulleys 549 and 550, and thereafter, a proximal portion of the wire 507 is attached to a linear actuator 537. In addition, a distal end portion of the wire 508 is fixed to the side surface of the rod-shaped rotational movement unit 520, passed through the lower surface side of the movement unit 510 via a through insertion hole drilled in the movement unit 510, and further wound around direction changing pulleys 551 and 552, and thereafter, a proximal portion of the wire 508 is attached to a linear actuator 538.

FIG. 6 illustrates a state in which the rotational movement unit 520 is viewed from above. When the wires 505 and 506 are pulled by the linear actuators 535 and 536, and the wires 507 and 508 are extended by the linear actuators 537 and 538 so as to balance with the pulling of the wires 505 and 506, the rotational movement unit 520 rotates about the Y-axis on the connecting portion 521 with respect to the movement unit 510 (or in the XYZ coordinate system), and falls in a positive X-direction. In addition, when the wires 507 and 508 are pulled by the linear actuators 537 and 538, and the wires 505 and 506 are extended by the linear actuators 535 and 536 so as to balance with the pulling of the wires 507 and 508, the rotational movement unit 520 rotates about the Y-axis on the connecting portion 521 and falls in a negative X-direction.

Meanwhile, when the wires 505 and 507 are pulled by the linear actuators 535 and 537, and the wires 506 and 508 are extended by the linear actuators 536 and 538 so as to balance with the pulling of the wires 505 and 507, the rotational movement unit 520 rotates about the X-axis on the connecting portion 521 with respect to the movement unit 510 (or in the XYZ coordinate system), and falls in a negative Y-direction. In addition, when the wires 506 and 508 are pulled by the linear actuators 536 and 538, and the wires 505 and 507 are extended by the linear actuators 535 and 537 so as to balance with the pulling of wires 5062 and 508, the rotational movement unit 520 rotates about the X-axis on the connecting portion 521 and falls in a positive Y-direction.

As described above, the parallel wire devices 100 to 500 illustrated in FIGS. 1 to 5 can translate the movement units 110, 210, ... supported by a plurality of parallel wires, and rotate the rotational movement units 120, 220, ... attached to the movement units 110, 210, .... Then, while the plurality of wires attached to the movement units 110, 210, ... includes parallel wires for translation and parallel wires for rotation, the wires do not interfere with each other at any of a time of translation, a time of rotation, or a time of performing translation and rotation at the same time.

In addition, in each of the parallel wire devices 100 to 500 illustrated in FIGS. 1 to 5, the linear actuators for translation and for rotation are all installed at the proximal portions of the respective wires. Hence, no driving actuators are mounted at all in the movement units 110, 210, ... translated and rotated by the parallel wires, so that the movement units 110, 210, ... can be fabricated in small size and with light weight. Because of the light weight, the movement units 110, 210, ... can be translated or rotated by relatively small pulling forces of the wires. In addition, even in a case where an accident in which the movement units 110, 210, ... fall occurs, damage can be minimized because of the small size and the light weight.

In the embodiments of the parallel wire devices 100 to 500 illustrated in FIGS. 1 to 5, the movement units 110, 210, ... have two degrees of freedom for translation in the XY plane. However, degrees of translational freedom in three directions can be realized by further adding two wires for pulling in a Z-direction. In addition, in the embodiments of the parallel wire devices 100 to 400 illustrated in FIGS. 1 to 4, the rotational movement units 120, 220, ... that can rotate only about one axis are attached to the movement units 110, 210, ..., and each have a degree of rotational freedom about one axis. However, two or more degrees of rotational freedom can also be realized by providing the rotational movement units 120, 220, ... with degrees of rotational freedom about two axes or more, and adding two wires for rotating the rotational movement units 120, 220, ... about each axis. For example, it suffices to mount a parallel link device on a movement unit capable of translation by parallel wires, and add two wires for driving each link.

Meanwhile, wires can generate only tension. It is known in the art that at least seven wires are therefore necessary to operate an object having six degrees of freedom to a freely-selected position by only wires (see PTL 4, for example). That is, when there are at least seven wires, the position (angle) of the target object can be controlled. In addition, 12 wires are necessary to completely control the six degrees of freedom including both the position (angle) and force (torque).

Further, two wires are necessary to drive the rotational movement unit 120 attached to the movement unit 110 as in the parallel wire device 100 according to the present embodiment, and two wires are necessary for each degree of freedom in a case where the rotational movement unit 520 has two or more degrees of rotational freedom as in the parallel wire device 500. Hence, a parallel wire device realizing the translation of a movement unit and the rotation of a rotational movement unit as in the present embodiment needs a minimum of 11 wires and a maximum of 16 wires.

When the number of wires is increased, the arrangement of the wires becomes complex, and the number of driving devices such as linear actuators pulling the wires is increased. Thus, a total weight is increased, and a cost is also increased. In contrast, a mechanism for reducing the number of wires may be introduced into a parallel wire device. For example, one of a minimum of seven wires for driving the parallel wire device can be used in a shared manner by using a difference between changes in the length of two wires for the rotation of a rotational movement unit and using a sum of the changes in the length for the translation of a movement unit. As described above, in the parallel wire device 400 illustrated in FIG. 4, the wires 405 and 406 for the rotation of the rotational movement unit 420 are also used as wires for the translation of the movement unit 410. The rotational movement unit 420 is rotated by using a difference between changes in the length of the wire 405 and the wire 406, and the movement unit 410 is translated by a sum of the changes in the length of the wire 405 and the wire 406.

The parallel wire devices 100 to 500 illustrated in FIGS. 1 to 5 have effects as follows.

(1) A wide rotational movable range can be achieved while low inertia intrinsic in the parallel wires is achieved.
(2) A wide translational movable range intrinsic in the parallel wires can be provided.
(3) The number of wires and actuators for driving the wires can be reduced by partially sharing the wires for translation and the wires for rotation.
(4) In a case where a universal joint is used at points of attachment of the wires, the attachment points are changed easily, a rotational center is not shifted according to the orientations of the wires, and an error from a model can be suppressed.

### Second Embodiment

FIG. 7 and FIG. 8 illustrate an example in which a parallel wire system is applied in a master device 700 of a master-slave system. However, FIG. 7 illustrates a state in which the master device 700 operated by an operator is obliquely viewed from the front of the operator, and FIG. 8 illustrates a state in which the master device 700 being operated by the operator is overlooked. Suppose that the master device 700 performs two-way communication with a slave device not illustrated, and that a bilateral system, for example, is used to operate a slave from a master and simultaneously feed back the state of the slave to the master.

A main body of the master device 700 is a box-shaped structure that opens in an upper surface thereof. A plurality of wires is extended in parallel from the side surface of the box to the inside of the box. Then, these wires support each of a controller 701L for a left hand and a controller 701R for a right hand in the air. In addition, a plurality of linear actuators that pulls the respective wires on proximal sides is attached to the master device 700.

As illustrated in FIG. 7 and FIG. 8, the operator can operate the controller 701L for the left hand and the controller 701R for the right hand in a state of placing the left and right hands in the box. As will be described later, the controllers 701L and 701R include a gripping force sense presenting device, and the operator operates the controllers 701L and 701R while gripping the gripping force sense presenting devices by the respective left and right hands. Neither of the controllers 701L and 701R includes a power source such as a linear actuator for pulling a wire or the like, and each of the controllers 701L and 701R can therefore be configured in small size, with light weight, and with low inertia, and provide the operator with good operability. The controller 701L for the left hand and the controller 701R for the right hand basically have a bilaterally symmetric shape and a bilaterally symmetric structure. In the following, description will be made mainly of the controller 701 for the right hand.

The controller 701 includes: a controller main body unit that is translated in a three-dimensional space by parallel wires, and a rotational movement unit that is attached so as to be rotatable about at least one axis with respect to a controller main body, and is rotated by parallel wires. In other words, the parallel wires supporting the controller 701 in the air include parallel wires for translating the controller main body and parallel wires for rotating the rotational movement unit. In addition, in FIG. 7 and FIG. 8, the wires used to translate the main body unit of the controller 701 are depicted by solid lines, and the wires used to rotate the rotational movement unit within the main body are depicted by broken lines. However, details of a mechanism for translating and rotating the controller 701 will be described later. The wires are pulled by linear actuators at proximal portions of the respective wires.

The proximal portion of each of the wires is oriented in a translational direction of the corresponding linear actuator, while the distal end portion of each of the wires is oriented in an operating direction of a target object to be translated or rotated. Each of the wires is wound around one or two or more pulleys (not illustrated in FIG. 7 nor FIG. 8) at an intermediate point(s) and folded back or changed in direction as appropriate, and each of the wires is arranged such that the wires do not interfere with each other.

The wires used in the master device 700 can be fabricated by using, for example, a metallic string (a wire rope including a stranded wire manufactured of stainless steel or the like) or a chemical fiber. The metallic string has an advantage of being not elongated easily. In addition, while there is a risk of the chemical fiber being elongated easily, the chemical fiber has an advantage of being conforming easily. In addition, not all of the wires to be used necessarily needs to uniformly include an identical material. Generally, a tension up to approximately 10 kgf is expected to act on the wires. However, a maximum instantaneous tension may not be limited to this value.

FIG. 9, FIG. 10, and FIG. 15 illustrate, in enlarged dimension, the controller 701 in the master device 700. Incidentally, for the convenience of description, in FIG. 9 and FIG. 10, XYZ axes are set as illustrated in the figures. The controller 701 illustrated in the figures includes: a controller main body unit that is translated in a three-dimensional space by parallel wires; and a rotational movement unit that is attached so as to be rotatable about at least one axis with respect to the controller main body, and is rotated by parallel wires.

The controller main body unit is formed by integrated three plates, that is, a bottom plate portion 801 having a Z-axis direction as a normal, a side wall portion 802 having a Y-axis direction as a normal, and a side wall portion 803 having an X-axis direction as a normal.

The bottom plate portion 801 may have four universal joints 811 to 814 for attaching a plurality of wires in parallel. When parallel wires for translation are attached (not illustrated in FIG. 9 nor FIG. 10) to the respective universal joints 811 to 814, and the bottom plate portion 801 is pulled, the controller 701 is supported so as to float in the air within the box of the master device 700, as illustrated in FIG. 7 and FIG. 8, and the controller 701 can be translated in each of the XYZ axial directions.

Incidentally, the universal joints 811 to 814 are joints that change in angle freely, and are used to make a rotational center fixed at all times when the universal joints 811 to 814 are pulled by the respective wires. That is, an angle at which each wire is incident on the bottom plate portion 801 changes according to a position to which the bottom plate portion 801 is translated. However, the universal joints 811 to 814 maintain the rotational center position of the wires at all times, and thereby suppress an error from an ideal model of a parallel wire mechanism and also contribute to a reduction in damage to the wires. In addition, when the universal joints are used, attachment points can be changed by screws, and it is therefore easy to change the attachment points without damaging the wires.

The rotational movement unit attached to the main body unit of the controller 701 is a parallel link structure with two degrees of freedom, the parallel link structure including a first serial link 851 and a second serial link 852. In addition, this parallel link is mounted with a gripping force sense presenting device 870 that the operator grips by hand and operates directly. Then, this parallel link is configured to be housed within a space of the controller main body unit including the bottom plate portion 801 and the two side wall portions 802 and 803.

A pulley 821 rotatable about a Y-axis is attached to the inner wall surface of the side wall portion 802. In addition, the first serial link 851 rotates about the Y-axis integrally with the pulley 821. Then, respective distal end sides of two wires 861 and 862 are wound around the periphery of the pulley 821 so as to be in opposite directions from each other.

Other end sides of these wires 861 and 862 are released to the outside of the controller main body unit via two respective insertion holes 803a and 803b drilled in the side wall portion 803, and are pulled by respective linear actuators (not illustrated in FIG. 9 nor FIG. 10). Hence, the first serial link 851 is rotated in a clockwise direction about the Y-axis integrally with the pulley 821 by pulling the wire 861 from the other end side and extending the wire 862 so as to balance with the pulling of the wire 861. In addition, the first serial link 851 is rotated in a counterclockwise direction about the Y-axis integrally with the pulley 821 by pulling the wire 862 from the other end side and extending the wire 861 so as to balance with the pulling of the wire 862.

A pulley 822 rotatable about an X-axis is attached to the inner wall surface of the side wall portion 803. In addition, the second serial link 852 rotates about the X-axis integrally with the pulley 822. Then, respective distal end sides of two wires 863 and 864 are wound around the periphery of the pulley 822 so as to be in opposite directions from each other.

Other end sides of these wires 863 and 864 are released to the outside of the controller main body unit via two respective insertion holes 802a and 802b drilled in the side wall portion 802, and are pulled by respective linear actuators (not illustrated in FIG. 9 nor FIG. 10). Hence, the second serial link 852 is rotated in a clockwise direction about the X-axis integrally with the pulley 822 by pulling the wire 863 from the other end side and extending the wire 864 so as to balance with the pulling of the wire 863. In addition, the second serial link 852 is rotated in a counterclockwise direction about the X-axis integrally with the pulley 822 by pulling the wire 864 from the other end side and extending the wire 863 so as to balance with the pulling of the wire 864.

Here, as is also understood from FIG. 9, one pair of the insertion holes 802a and 802b on the side wall portion 802 side and one pair of the insertion holes 803a and 803b on the side wall portion 803 side are drilled with height differences (that is, such that the positions thereof in the Z-direction do not coincide with one another). Hence, the wires 861 and 862 inserted through the insertion holes 803a and 803b and the wires 863 and 864 inserted through the insertion holes 802a and 802b pass on different planes, and can pull the respective pulleys 821 and 822 for rotation so as not to interfere with one another.

The pulley 822 is disposed such that the axis of rotation of the pulley 822 is orthogonal to that of the pulley 821 that rotates about the Y-axis. In addition, a parallel link including the first serial link 851 and the second serial link 852 realizes degrees of rotational freedom about two axes orthogonal to each other. For example, the first serial link 851 and the second serial link 852 can be rotated by approximately ±80 degrees about the respective rotational axes. Hence, two degrees of rotational freedom can be imparted to the gripping force sense presenting device 870 mounted on the parallel link. Further, a flat type motor 881 may be mounted. A third degree of freedom that enables a wide rotation of ±90 degrees or more about the rotational axis of the motor 881 can be imparted to the gripping force sense presenting device 870. The use of a flat type motor as the motor 881 can realize a simple structure that does not easily collide with surroundings at a time of rotation.

As is understood from FIGS. 7 to 10, the master device 700 drives the controller 701 by the parallel wire system. The parallel wires have low inertia. In addition, according to the parallel wire mechanism of translating the controller 701 in three directions by the wires for translation and rotating the gripping force sense presenting device 870 within the controller 701 about two axes by the wires for rotation, a force can be presented to the operator, and the state of the slave is fed back to the master at the same time as the slave is operated from the master. Thus, bilateral control can be realized suitably.

The gripping force sense presenting device 870 is a UI (User Interface) mounted in the controller 701, and gripped by hand and operated by the operator. FIG. 7 and FIG. 8 illustrate a state in which the operator is operating the gripping force sense presenting devices respectively mounted in the left and right controllers 701L and 701R by a left hand and a right hand. Incidentally, a six-axis force sensor (not illustrated) may be mounted on the proximal side of the gripping force sense presenting device 870. An order in which the six-axis force sensor and the motor 881 are arranged is optional. There is a case where it is better to dispose the force sensor closer to the proximal side than the motor 881 in consideration of cable wiring design.

The technology disclosed in PTL 3, for example, can be applied to the gripping force sense presenting device 870. A configuration of the gripping force sense presenting device 870 will be briefly described in the following.

FIGS. 11 to 13 illustrate a state in which the operator is using the gripping force sense presenting device 870. The gripping force sense presenting device 870 includes a rod-shaped main body portion 1101, a link portion 1104 supported by a bearing portion 1102 in the vicinity of a distal end of the main body portion 1101 so as to be rotatable about a rotational center 1103, a finger contact portion 1105 on the upper surface of a distal end of the link portion 1104, and a rail portion 1106 on the lower surface of the distal end of the link portion 1104.

The operator can hold the gripping force sense presenting device 870 so as to sandwich the main body portion 1101 between a thumb and a middle finger. In addition, the link portion 1104 can be operated so as to be opened and closed with respect to the main body portion 1101 by making an index finger abut against the finger contact portion 1105 and further pushing in the index finger. In addition, the rail portion 1106 appears from the main body portion 1101 and disappears with the opening and closing operation of the link portion 1104.

Though not illustrated, housed within the main body portion 1101 are an encoder that measures the rotational angle of the link portion 1104, an actuator that applies a load to the opening and closing operation of the link portion 1104, and a transmitting mechanism that transmits a driving force of the actuator. See PTL 3 for details.

In a case where the master-slave system is applied to a medical robot that performs a surgical operation (for example, a surgical operation using an endoscope, a microscope, or a catheter) or the like, the controller 701L for the left hand and the controller 701R for the right hand should be installed as a surgeon console for operating a medical instrument attached on the slave side from the master side. In addition, when ergonomics is taken into consideration, the controller 701L for the left hand and the controller 701R for the right hand are preferably attached in attitudes in which the operator easily operates the controller 701L for the left hand and the controller 701R for the right hand by respective left and right arms and hands. When the controller 701L for the left hand and the controller 701R for the right hand are each attached at an appropriate angle, the operator operates the controller 701L for the left hand and the controller 701R for the right hand easily, can perform work with high accuracy, and does not feel the fatigue of the arms and the hands easily. The operator can therefore endure work for a long period of time.

In general, a human does not become tired easily in a state in which a hand goes downward from upward. Accordingly, in the example illustrated in FIG. 7 and FIG. 8, assuming a state in which the left and right hands of the operator go downward from upward, each of the controller 701L for the left hand and the controller 701R for the right hand is attached such that the gripping force sense presenting device 870 is oriented upward.

In addition, when an elbow is bent to a certain extent, the person performs operation more easily, can respond instantly, and does not hurt the elbow easily. Ergonomically, an attitude is preferable in which fingers of the left and right hands of the operator respectively go from the left and right outsides to the front of the body. Accordingly, in the example illustrated in FIG. 7 and FIG. 8, the controller 701L for the left hand and the controller 701R for the right hand are each disposed such that the pulleys 821 and 822 supported by the side wall portions 802 and 803 are on the inside of both hands, and such that the gripping force sense presenting devices 870 are oriented to the left and right outsides. It can also be said that the rotational movement unit is disposed so as to be on the inside of the hand of the operator when the parallel wire device is installed and used in front of the operator. Then, the operator can grip the gripping force sense presenting device 870 in a state in which the elbows of the left and right arms are bent to a certain extent and in an attitude in which the left and right hands each go from the outside to the front of the body. Thus, the operator performs operation easily, can respond instantly, does not hurt the elbow easily, and can proceed with an operation procedure smoothly.

According to the arrangement of the controllers 701L and 701R as illustrated in FIG. 7 and FIG. 8, the operator can operate the controllers 701L and 701R while using a wide movable range intrinsically possessed by the arms of the human. Conversely, when the controllers 701L and 701R are arranged in directions in which it is difficult to orient the hands of the human without ergonomics being taken into consideration, the operator can use only a part of the movable range possessed by the arms of the operator for the operation of the controllers 701L and 701R, and therefore becomes fatigued easily and hurts the elbows easily.

### Third Embodiment

The rotational movement unit attached to the main body unit of the controller 701 in the embodiment illustrated in FIGS. 7 to 10 is a two-axis parallel link structure and is not limited to this. It suffices to mount a parallel link device with three axes or more in the main body unit of the controller 701 and drive each link by two wires.

For example, a spherical parallel link device having three degrees of rotational freedom, the spherical parallel link device being referred to also as an "Agile eye," can also be mounted in the main body unit of the controller 701. The spherical parallel link device is a parallel link device in which each link is configured to move on a spherical surface having a common center.

FIG. 14 illustrates an example of a configuration of the spherical parallel link device. The spherical parallel link device 1400 illustrated in the figure includes a base portion 1401, three serial links attached in parallel to the base portion 1401, and an end effector portion 1402 supported by distal end portions of these three serial links.

In the spherical parallel link device 1400 illustrated in the figure, the three parallel serial links are of a substantially identical configuration. The three parallel serial links each include a proximal portion 1411 attached with a degree of rotational freedom about one axis to the base portion 1401, a driving link 1412 that rotates integrally with the proximal portion 1411, and a driven link 1413 that is coupled to a distal end portion of the driving link 1412 and supports the end effector portion 1402 at a distal end portion of the driven link 1413.

The proximal portion 1411 of each link is formed as a pulley, for example. Two wires 1421 and 1422 are wound around the periphery of the proximal portion 1411 in opposite directions from each other. The proximal portion 1411 and the driving link 1412 can be rotation-driven by pulling one of the wires 1421 and 1422 and extending the other. Then, the three-dimensional attitude of the end effector portion 1402 with respect to the base portion 1401 can be changed by synchronously driving the three serial links.

The spherical parallel link device 1400 is characterized by having a wide movable range and being driven at high speed and high acceleration. When a camera is mounted on the end effector portion 1402, for example, the camera has a wider angle of view than an eye of a human, and a line of sight can be changed at higher speed and high acceleration than an eye of a human.

### Fourth Embodiment

A range of application of the parallel wire device disclosed in the present specification is not limited to a controller of a master device. For example, the parallel wire device as described above may be applied for translation and rotation of a remote operation target such as a manipulator on the slave device side or the like.

In addition, the range of application of the parallel wire device disclosed in the present specification is not limited to the master-slave system.

For example, when a moving projector is floated in the air by the parallel wire device disclosed in the present specification, the moving projector can be translated in a three-dimensional space, and further a video projecting direction can be changed in a wide movable range at each position to which the moving projector is moved. Specifically, the rotational movement unit including the parallel link structure as illustrated in FIG. 14 is mounted on the movement unit, and the projector is attached to the parallel link. The pulling of the wire can translate the projector in the three-dimensional space, and change the projecting direction at each movement position. It is therefore possible to realize a moving projector having a wide translational movable range and a wide rotational movable range.

In addition, when photographing equipment such as a camera or the like is floated in the air by the parallel wire device disclosed in the present specification, the camera can be translated in the three-dimensional space to change a viewpoint position, and further the sight line direction of the camera can be changed in a wide movable range at each viewpoint position to which the camera is moved. In a case where a ceiling suspension type camera is used in a stadium in which a ball game such as soccer, athletic sports, or the like is performed, for example, a plurality of parallel wires is provided on a ceiling or in the air in the sporting venue, and the pulling of the wires can move the ceiling suspension type camera or adjust a pan or a tilt in a wide range. Specifically, the rotational movement unit including the parallel link structure as illustrated in FIG. 14 is mounted on the movement unit, and the ceiling suspension type camera is attached to the parallel link. The pulling of the wires can translate the viewpoint position of the camera in the three-dimensional space, and change the sight line direction of the camera at each movement position. It is therefore possible to realize a moving camera having a wide translational movable range and a wide rotational movable range.

In a case where the equipment such as the moving projector, the camera, or the like is floated in the air by the parallel wire device disclosed in the present specification, linear actuators for driving the wires are arranged in the vicinity of the proximal portions of the respective wires. Thus, the movement unit mounted with the equipment can be fabricated in small size and with light weight. Hence, the equipment can be translated or rotated with a relatively small pulling force. In addition, even in a case where an accident in which the movement unit mounted with the equipment falls occurs, damage can be minimized because of the small size and the light weight.

Incidentally, it is to be understood that the parallel wire device applied to the master device 700 illustrated in FIGS. 7 to 10 has effects similar to those of the parallel wire device described in the first embodiment. The parallel wire device has low inertia, and has a wide translational movable range and a wide rotational movable range. When the controller 701 is driven by using such a parallel wire device, a force can be presented to the operator, and the state of the slave is fed back to the master at the same time as the slave is operated from the master. Thus, bilateral control can be realized suitably.

### [Industrial Applicability]

The technology disclosed in the present specification has been described above in detail with reference to particular embodiments. However, it is obvious that those skilled in the art can make modifications and substitutions in the embodiments without departing from the spirit of the technology disclosed in the present specification.

The parallel wire device to which the technology disclosed in the present specification is applied can be used for the translation and rotation of the controller on the master device side and for the translation and rotation of the operation target such as a manipulator on the slave device side in the master-slave system, for example. The applications of the parallel wire device to which the technology disclosed in the present specification is applied are not limited to the master-slave system. In addition, various equipment including a moving projector and a camera can be floated in the air and translated and rotated by using the parallel wire device to which the technology disclosed in the present specification is applied.

In short, the technology disclosed in the present specification has been described in a form of illustration, and the contents described in the present specification are not to be construed in a limited manner. In order to determine the gist of the technology disclosed in the present specification, claims are to be considered.

Incidentally, the technology disclosed in the present specification can also have the following configurations.
(1) A parallel wire device including:
   a movement unit;
   a rotational movement unit attached to the movement unit so as to be rotatable about at least one axis;
   a first parallel wire configured to translationally drive the movement unit; and
   a second parallel wire configured to rotationally drive the rotational movement unit.
(2) The parallel wire device according to the above (1), in which
   the rotational movement unit has two or more degrees of rotational freedom with respect to the movement unit, and
   the second parallel wire includes a parallel wire configured to rotationally drive the rotational movement unit about one axis and a parallel wire configured to rotationally drive the rotational movement unit about another axis.
(3) The parallel wire device according to the above (1), in which
   the rotational movement unit includes a serial link, and
   the second parallel wire includes a parallel wire configured to drive the serial link.
(4) The parallel wire device according to the above (1), in which
   the rotational movement unit includes a parallel link, and
   the second parallel wire includes parallel wires configured to drive respective links constituting the parallel link.
(5) The parallel wire device according to the above (1), in which
   the second parallel wire includes two wires configured to rotationally drive the rotational movement unit about one axis.
(6) The parallel wire device according to the above (1), in which
   the second parallel wire includes a wire used for translationally driving the movement unit in a shared manner.
(7) The parallel wire device according to the above (6), in which
   the second parallel wire includes two wires configured to rotationally drive the rotational movement unit by using a difference between changes in length, and translationally drive the movement unit by using a sum of changes in length.
(8) The parallel wire device according to the above (4), further including:
   an actuator configured to drive a structure attached to a distal end of the parallel link.
(9) The parallel wire device according to the above (2), in which
   the parallel wire configured to rotationally drive the rotational movement unit about one axis and the parallel wire configured to rotationally drive the rotational movement unit about the other axis are arranged so as to pass on different planes and so as not to interfere with each other.
(10) The parallel wire device according to any one of the above (1) to (9), in which
   when the parallel wire device is provided and used in front of an operator, the rotational movement unit is disposed so as to be on an inside of a hand of the operator.
(11) A parallel wire system including:
   a movement unit;
   a rotational movement unit attached to the movement unit so as to be rotatable about at least one axis;
   a first parallel wire configured to translationally drive the movement unit;
   a second parallel wire configured to rotationally drive the rotational movement unit; and
   a plurality of actuators configured to drive respective wires included in the first parallel wire and the second parallel wire.
(12) An operating device for a medical robot of a master-slave system, the operating device being for operating a medical instrument attached to a slave side from a master side, the operating device including:
   a movement unit;
   a rotational movement unit attached to the movement unit so as to be rotatable about at least one axis;
   an operating unit for an operator, the operating unit being attached to the rotational movable unit;
   a first parallel wire configured to translationally drive the movement unit; and
   a second parallel wire configured to rotationally drive the rotational movement unit.
(12-1) A medical robot of a master-slave system, the medical robot including an operating device for the medical robot of the master-slave system, the operating device being on a master side that operates a medical instrument attached to a slave side, the operating device including:
   a movement unit;
   a rotational movement unit attached to the movement unit so as to be rotatable about at least one axis;
   an operating unit for an operator, the operating unit being attached to the rotational movable unit;
   a first parallel wire configured to translationally drive the movement unit; and
   a second parallel wire configured to rotationally drive the rotational movement unit.
(13) A mobile projecting device including:
   a movement unit;
   a rotational movement unit attached to the movement unit so as to be rotatable about at least one axis;
   a projecting device attached to the rotational movable unit;
   a first parallel wire configured to translationally drive the movement unit; and
   a second parallel wire configured to rotationally drive the rotational movement unit.
(14) A mobile photographing device including:
   a movement unit;
   a rotational movement unit attached to the movement unit so as to be rotatable about at least one axis;
   a camera attached to the rotational movable unit;
   a first parallel wire configured to translationally drive the movement unit; and
   a second parallel wire configured to rotationally drive the rotational movement unit.

### [Reference Signs List]

- 100: Parallel wire device
- 101 to 106: Wire
- 110: Movement unit
- 120: Rotational movement unit
- 131 to 136: Linear actuator
- 141 to 148: Direction changing pulley
- 200: Parallel wire device
- 201 to 206: Wire
- 210: Movement unit
- 220: Rotational movement unit
- 231 to 236: Linear actuator
- 241 to 248: Direction changing pulley
- 300: parallel wire device
- 301 to 306: Wire
- 310: Movement unit
- 320: Rotational movement unit
- 331 to 336: Linear actuator
- 341 to 348: Direction changing pulley
- 351, 352: Direction changing pulley
- 400: Parallel wire device
- 401, 404, 405, 106: Wire
- 410: Movement unit
- 420: Rotational movement unit
- 431, 434, 435, 436: Linear actuator
- 441, 444 to 148: Direction changing pulley
- 500: Parallel wire device
- 101 to 106: Wire
- 510: Movement unit
- 520: Rotational movement unit
- 521: Connecting portion
- 531 to 538: Linear actuator
- 541 to 552: Direction changing pulley
- 700: Master device
- 701: Controller
- 801: Bottom plate portion
- 802: Side wall portion
- 802a, 802b: Insertion hole
- 803: Side wall portion
- 803a, 803b: Insertion hole
- 811 to 814: Universal joint
- 821, 822: Pulley
- 851: First serial link
- 852: Second serial link
- 861 to 864: Wire
- 870: Gripping force sense presenting device
- 881: Motor
- 1101: Main body portion
- 1102: Bearing portion
- 1103: Rotational center
- 1104: Link portion
- 1105: Finger contact portion
- 1106: Rail portion
- 1400: Spherical parallel link device
- 1401: Base portion
- 1402: End effector portion
- 1411: Proximal portion (pulley)
- 1412: Driving link
- 1413: Driven link
- 1421, 1422: Wire

## Claims

1. A parallel wire device comprising:
a movement unit;
a rotational movement unit attached to the movement unit so as to be rotatable about at least one axis;
a first parallel wire configured to translationally drive the movement unit; and
a second parallel wire configured to rotationally drive the rotational movement unit.

2. The parallel wire device according to claim 1, wherein
the rotational movement unit has two or more degrees of rotational freedom with respect to the movement unit, and
the second parallel wire includes a parallel wire configured to rotationally drive the rotational movement unit about one axis and a parallel wire configured to rotationally drive the rotational movement unit about another axis.

3. The parallel wire device according to claim 1, wherein
the rotational movement unit includes a serial link, and
the second parallel wire includes a parallel wire configured to drive the serial link.

4. The parallel wire device according to claim 1, wherein
the rotational movement unit includes a parallel link, and
the second parallel wire includes parallel wires configured to drive respective links constituting the parallel link.

5. The parallel wire device according to claim 1, wherein
the second parallel wire includes two wires configured to rotationally drive the rotational movement unit about one axis.

6. The parallel wire device according to claim 1, wherein
the second parallel wire includes a wire used for translationally driving the movement unit in a shared manner.

7. The parallel wire device according to claim 6, wherein
the second parallel wire includes two wires configured to rotationally drive the rotational movement unit by using a difference between changes in length, and translationally drive the movement unit by using a sum of changes in length.

8. The parallel wire device according to claim 4, further comprising:
an actuator configured to drive a structure attached to a distal end of the parallel link.

9. The parallel wire device according to claim 2, wherein
the parallel wire configured to rotationally drive the rotational movement unit about one axis and the parallel wire configured to rotationally drive the rotational movement unit about the other axis are arranged so as to pass on different planes and so as not to interfere with each other.

10. The parallel wire device according to claim 1, wherein
when the parallel wire device is provided and used in front of an operator, the rotational movement unit is disposed so as to be on an inside of a hand of the operator.

11. A parallel wire system comprising:
a movement unit;
a rotational movement unit attached to the movement unit so as to be rotatable about at least one axis;
a first parallel wire configured to translationally drive the movement unit;
a second parallel wire configured to rotationally drive the rotational movement unit; and
a plurality of actuators configured to drive respective wires included in the first parallel wire and the second parallel wire.

12. An operating device for a medical robot of a master-slave system, the operating device being for operating a medical instrument attached to a slave side from a master side, the operating device comprising:
a movement unit;
a rotational movement unit attached to the movement unit so as to be rotatable about at least one axis;
an operating unit for an operator, the operating unit being attached to the rotational movable unit;
a first parallel wire configured to translationally drive the movement unit; and
a second parallel wire configured to rotationally drive the rotational movement unit.

13. A mobile projecting device comprising:
a movement unit;
a rotational movement unit attached to the movement unit so as to be rotatable about at least one axis;
a projecting device attached to the rotational movable unit;
a first parallel wire configured to translationally drive the movement unit; and
a second parallel wire configured to rotationally drive the rotational movement unit.

14. A mobile photographing device comprising:
a movement unit;
a rotational movement unit attached to the movement unit so as to be rotatable about at least one axis;
a camera attached to the rotational movable unit;
a first parallel wire configured to translationally drive the movement unit; and
a second parallel wire configured to rotationally drive the rotational movement unit.
